# EUROPEAN PATENT APPLICATION

(11) **EP 2 067 494 A1**
(43) Date of publication of application: **10.06.2009**
(21) Application number: 07023464.6
(22) Date of filing: 04.12.2007
(51) Int. Cl.: A61L 27/34, A61L 27/54, A61L 31/10, A61L 31/16, A61F 2/06

(54) **Sheet or tubular structure consisting of elastic biocompatible material and its use**

(71) Applicant: Charité - Universitätsmedizin Berlin, 10117 Berlin (DE)
(72) Inventor: Duda, Georg, 12209 Berlin (DE); Manav, Metha, 10437 Berlin (DE); Strube, Patrick, 13187 Berlin (DE)
(74) Representative: Zwicker, Jörk

(57) **Abstract**

The present invention relates to a sheet or tubular structure consisting of biocompatible material, which is elastic and comprises at least one biologically active substance in at least one region and to a sheet or tubular structure, which comprises at least one biologically active substance release-modifying agent in at least one region as well as an implant covered at least partially by the sheet or tubular structure of the invention. The present invention further provides a method and an apparatus for producing, and preferably customizing and/or optimizing, the sheet or tubular structure of the invention. The sheet or tubular structure of the invention can be customized/optimized before implantation in the operating room.

## Description

The present invention relates to a sheet or tubular structure consisting of biocompatible material, which is elastic and comprises at least one biologically active substance in at least one region and to a sheet or tubular structure, which comprises at least one biologically active substance release-modifying agent in at least one region as well as an implant covered at least partially by the sheet or tubular structure of the invention. The present invention further provides a method and an apparatus for producing, and preferably customizing and/or optimizing, the sheet or tubular structure of the invention.The sheet or tubular structure of the invention can be customized/optimized before implantation in the operating room.

### Background of the Invention

Significant areas of application for bone implants include, for example, the closing of large bone defects associated with comminuted fractures as well as the attachment of bone fragments, the filling of bone defects resulting from bone cysts and after removal of bone tumors, the filling of voids caused by chronic osteomyelitis, applications associated with injury of and/or material loss on alveolis and jaw bones and joints. The importance of biocompatible implants including bone implants, in particular in the areas of orthopedics, traumatology, cranial, dental and facial surgery, and orthodontics continues to increase.

When repairing a missing, broken or diseased portion of bone, implants can be used. Implants can be, e.g. intramedullary implants, joint replacements (e.g., a hip or knee implant), or tooth implants. Implants being used today are fabricated from a variety of materials including metal, polymer and ceramics. Implant materials that are routinely used comprise, e.g. titanium (Ti) in its commercially pure state or alloys of titanium with aluminum and vanadium, or cobalt chrome (Co--Cr) which may also contain molybdenum (Mo). Mo is added to improve the wear resistance properties of the material, an important consideration when the implant is used to replace articulating surfaces.

In some applications polymer substance may have superior qualities over the metal implants, since polymers can e.g. minimizes the stress shielding effect created by a metal implant, thus, leading to a longer implant lifetime in the body. Polymer bone implants can comprise a thermoplastic polymer with an elastic modulus approximating the modulus of bone or a composite comprising a thermoplastic polymer and a reinforcing material, the composite also having an elastic modulus approximating the elastic modulus of bone.

To improve the properties of implant, e.g. to foster long term stabilization of the implant in bone, a porous coating is typically applied onto the surface of the implant. Ti or hydroxyapatite (HA) are two materials with good biocompatibility and/or biostimulating factors commonly used to create this porous coating. Ti is sintered onto the surface of the metal (e.g., Ti) implant in either a mesh of crimped wire or a random array of particulates. The HA is applied to the surface of the implant using plasma spraying techniques. The porous coating must have large enough pores to allow the bone cells to travel through and create a strong interlocking fixation by reconnecting with adjacent bone tissue throughout the mesh.

To further improve the properties of implants, e.g. to promote healing, drug substances have been coated to the surface of the implant. The bioactive drug substance can have the property of accelerating the healing process by having e.g. antibiotic, cytostatic and/or osteogenic properties (see e.g. Price, J. S. et al. (1996) J. Biomed Mater Res. 30(3):281-6, Catterall J.B. and Cawston, T.E. (2003) Arthritis Res Ther. 5(1):12-24 or Chu T.-M. C. et al. (2007) Biomaterials 28(3):459-67). Further information about bioactive drugs that can improve fracture healing and that may be used for implants are found e.g. in Schmidmaier G, et al., "Local application of growth factors IGF-I and TGF-b1 from a biodegradable poly(D,L-lactide) coating of osteosynthetic implants accelerate fracture healing", Bone, Apr 2001, pages 341-50; and further in: "Prophylaxis and treatment of implant-related infections by antibiotic-coated implants: a review", Injury, Volume 37, Issue 2, pages S105-S112 by G. Schmidmaier, M. Lucke, B. Wildemann, N. Haas, M. Raschke; and further in: Schmidmaier G, et al., "Long-term effects of local growth factor (IGF-I and TGF-1) treatment on fracture healing. A safety study for using growth factors", J Orthop Res., May 2004, pages 514-9.

Metal implants are not easily modified to comprise a drug release mechanism. Therefore, methods for coating implants have been tried, wherein the coats serve as drug carriers. These implant coats, however, fail as the coats are often too mechanically unstable during implant fixation. Additionally, the bioactive drug substances required by an individual patient can depend on many different factors. For example, systemic factors involved in the healing process are different in cases of old, young, diabetic, and smoking cases. It is, thus, also a severe deficit in above-mentioned implants that the type, amount, and location of the drug on the surface of the implant is determined during the pre-coating process and can not be modified by the physician to suit the patients needs. Therefore, depending on the case, it is generally not possible to use a combination of already approved drugs.

Thus, the pre-coated implants of the prior art do not allow customization of the drug or drugs delivered via the implant as required by the respective patient, patient specific customization, e.g. dose-modifications or combinations of drugs. Customization is very necessary as we see in many cases in fracture healing or in tumor implant cases, where patients are allergic to certain drugs or need a certain drug in excessive amounts. If the desired combination of drug type and drug amount were to be ordered for each patient from the manufacturer as a customized implant, the inventory costs for such implants would be exceedingly high. This would have strong negative implications on the economy of the healthcare system.

### SUMMARY OF THE INVENTION

The sheet or tubular structure consisting of biocompatible material of the present invention and its use in conjunction with implants provides several advantages over pre-coated prior art implants including flexibility, ease of use, and point-of-care individualization of implants. In particular the sheet or tubular structure of the present invention allow to vary the dose of the drug to be administered, to combine drugs, to combine drugs and cells, allow onsite customization and optimization of the drug delivery and/or to increase the selectivity of drug delivery. In a first aspect the present invention provides a sheet or tubular structure consisting of biocompatible material which is elastic and comprises at least one biologically active substance in at least one region.

In a further aspect the present invention relates to a sheet or tubular structure consisting of biocompatible material which is elastic and comprises at least one biologically active substance release-modifying agent in at least one region.

In a further aspect the present invention relates to an implant covered at least partially by a sheet or tubular structure consisting of the present invention.

In a further aspect the present invention relates to a method of producing the sheet or tubular structure of the invention comprising the step of contacting the sheet or tubular structure in at least one region with at least one biologically active substance and/or at least one biologically active substance release-modifying agent.

In a further aspect the present invention relates to a method of producing an enhanced implant comprising the steps of the above-stated method of the invention, and further comprising the step of at least partially covering an implant with the sheet or tubular structure.

In a further aspect the present invention relates to a sheet or tubular structure producible by the method of the present invention.

In a further aspect the present invention relates to an enhanced implant producible by the method of the invention.

In a further aspect the present invention relates to the use of a sheet or tubular structure of the invention and of an implant for the preparation of an enhanced implant, in particular for the treatment of a defective, fractured or lost bone, bone-structure or tooth or for the treatment of a disease-induced localized flow constriction in a patient.

In a further aspect the present invention relates to an apparatus for producing a sheet or tubular structure according to the invention comprising:
(i) a dispenser device (2) for dispensing one or more a sheets or tubular structures (1);
(ii) at least one reservoir (3) capable of containing a biologically active substance and/or a biologically active substance release-modifying agent; and
(iii) a release system (4) capable of releasing a biologically active substance and/or a biologically active substance release-modifying agent from at least one reservoir (3) onto the a sheet or tubular structure (1).

In a further aspect, the invention provides a method of treating a patient suffering from a disease or an ailment selected from the group consisting of a defective, fractured or lost bone, bone-structure or tooth, of a disease-induced localized flow constriction, of a heart disease, of chronic or acute pain, of a genetic disorder, of arthritis, of rheumatism of cancer of an inflammation and of an infection, wherein the method of treating a patient comprises the steps:
(i) using the apparatus according to the invention to produce a customized sheet or tubular structure according to the invention; wherein the sheet or tubular structure is preferably customized to meet the needs of the patient;
(ii) optionally at least partially covering an implant with the customized sheet or tubular structure; and
(iii) implanting said customized sheet or tubular structure or the implant which is at least partially covered with the customized sheet or tubular structure into the patient.

### DETAILED DESCRIPTION OF THE INVENTION

Before the present invention is described in detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Preferably, the terms used herein are defined as described in "A multilingual glossary of biotechnological terms: (IUPAC Recommendations)", Leuenberger, H.G.W, Nagel, B. and Klbl, H. eds. (1995), Helvetica Chimica Acta, CH-4010 Basel, Switzerland) and as described in "Pharmaceutical Substances: Syntheses, Patents, Applications" by Axel Kleemann and Jurgen Engel, Thieme Medical Publishing, 1999; the "Merck Index: An Encyclopedia of Chemicals, Drugs, and Biologicals", edited by Susan Budavari et al., CRC Press, 1996, and the United States Pharmacopeia-25/National Formulary-20, published by the United States Pharmcopeial Convention, Inc., Rockville Md., 2001.

Throughout this specification and the claims which follow, unless the context requires otherwise, the word "comprise", and variations such as "comprises" and "comprising", will be understood to imply the inclusion of a stated feature, integer or step or group of features, integers or steps but not the exclusion of any other feature, integer or step or group of integers or steps. In the following passages different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Several documents are cited throughout the text of this specification. Each of the documents cited herein (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention.

In the following, some definitions of terms frequently used in this specification are provided. These terms will, in each instance of its use, in the remainder of the specification have the respectively defined meaning and preferred meanings.

Any biologically active substance and/or biologically active substance release-modifying agent of the present invention may be admixed with a pharmaceutically acceptable diluent, excipient or carrier, or a mixture thereof. Even though the compounds of the present invention (including their pharmaceutically acceptable salts, esters and pharmaceutically acceptable solvates) can be used alone, they can be combined with a pharmaceutical carrier, excipient or diluent. The pharmaceutical compositions may be for human or animal usage in human and veterinary medicine. Examples of such suitable excipients for the various different forms of pharmaceutical compositions described herein may be found in the "Handbook of Pharmaceutical Excipients", 2nd Edition, (1994), Edited by A Wade and PJ Weller.

Acceptable carriers or diluents for therapeutic use are well known in the pharmaceutical art, and are described, for example, in Remington's Pharmaceutical Sciences, Mack Publishing Co. (A. R Gennaro edit. 1985). Examples of suitable carriers include lactose, starch, glucose, methyl cellulose, magnesium stearate, mannitol, sorbitol and the like. Examples of suitable diluents include alcohols, e.g. ethanol, glycerol, water and water-based buffers, e.g. phosphate buffer.

The biologically active substance and/or biologically active substance release-modifying agent of the present invention may comprise also a suitable binder(s), lubricant(s), suspending agent(s), coating agent(s), solubilising agent(s) and combinations thereof.

Examples of suitable binders include starch, gelatin, natural sugars such as glucose, anhydrous lactose, free-flow lactose, beta-lactose, corn sweeteners, natural and synthetic gums, such as acacia, tragacanth or sodium alginate, carboxymethyl cellulose and polyethylene glycol. Examples of suitable lubricants include sodium oleate, sodium stearate, magnesium stearate, sodium benzoate, sodium acetate, sodium chloride and the like. Also preservatives, stabilizers, and especially dyes may be provided in the biologically active substance and/or biologically active substance release-modifying agent of the present invention. Examples of preservatives include sodium benzoate, sorbic acid and esters of p-hydroxybenzoic acid. Antioxidants and suspending agents may be also used.

As used herein, a "region" of a sheet or tubular structure refers to a localized area of the sheet or tubular structure. A region onto which a biologically active substance and/or biologically active substance release-modifying agent has been applied may comprise the same concentration of biologically active substance and/or biologically active substance release-modifying agent on and/or in the sheet or tubular structure or may comprise different concentrations, e.g. a gradient, which may be formed by applying the biologically active substance and/or biologically active substance release-modifying agent to a small region and the ensuing distribution of the biologically active substance and/or biologically active substance release-modifying agent. Preferably, the "region" is defined by the medical practitioner, when applying the biologically active substance and/or biologically active substance release-modifying agent, e.g. at the point-of-care, as required by the patient. The application of the biologically active substance and/or biologically active substance release-modifying agent to separate regions that do not cover the entire surface of the sheet or tubular structure is preferable in cases wherein expensive biologically active substance and/or biologically active substance release-modifying agents are applied to the sheet or tubular structure. For example bone morphogenic factors may only be added to those regions of the sheet or tubular structure that will make contact with bone tissue once implanted. To aid the application of the biologically active substance and/or biologically active substance release-modifying agent to a particular region(s) a physiologically acceptable dye may be added to the biologically active substance and/or biologically active substance release-modifying agent. This will enhance the visualization of the region during and after application of the biologically active substance and/or biologically active substance release-modifying agent. The shape of the region is not particularly limited and is in part determined by the method of application of the biologically active substance and/or biologically active substance release-modifying agent. Preferably, the at least one region is localized on the outer-surface of the sheet or tubular structure. In case of a sheet like structure the outer surface is the surface, which will face the physiological environment once implanted and the inner surface will be the region facing the implant. In case of a tubular structure the region is preferably localized on the outer circumference of the tubular structure to face the physiological environment. It may, however, be preferred in certain embodiments to only or additionally apply the biologically active substance and/or biologically active substance release-modifying agent to the inner surface of the sheet or tubular structure. Depending on the biocompatible material that has been used for the production of the sheet or tubular structure a region may be defined both by the surface area to which the biologically active substance and/or biologically active substance release-modifying agent has been applied and the depth of penetration of the biologically active substance and/or biologically active substance release-modifying agent into the biocompatible material. For porous or filamentous biocompatible materials the regions may extend through the entire sheet or tubular structure and, thus, there will be a region comprising biologically active substance and/or biologically active substance release-modifying agent on both sides, i.e. the inner and the outer surface, of the sheet or tubular structure.

The term "elastic" as used in the present invention refers to the ability of sheet or tubular structure to be reversibly extended or plastically deformed in at least one direction, preferably in two directions, upon application of a force. In a tubular structure this extension may occur along the circumference, in a radial direction and/or along the longitudinal axis of the tubular biocompatible material. Preferably, the tubular structure has elasticity in radial direction and/or along the longitudinal axis of at least 5%, more preferably at least 10%, more preferably of at least 20%, 25%, 30%, 35%, 40%, 45%, 50%, 55%, 60%, 70%, 80%, 90, 100%, 120%, 140%, 160%, 180%, 200%, 300% or more. Similarly, the sheet structure preferably has elasticity along its length and/or width of at least 20%, 25%, 30%, 35%, 40%, 45%, 50%, 55%, 60%, 70%, 80%, 90, 100%, 120%, 140%, 160%, 180%, 200%, 300%400%, 500% or more. A tubular biocompatible material has an elasticity of at least 10%, if the circumference of the tubular material can be reversibly extended by at least 10%. Similarly, a segment of tubular biocompatible material of 10 cm length with an elasticity of at least 10% can be reversibly extended along its longitudinal axis by at least 1 cm. The elasticity of the sheet or tubular structure is not only dependent on the composition of the biocompatible material itself but also on the structure of the material. A material with low inherent elasticity like, e.g. polytetrafluoroethylene (PTFE) can be manufactured into a biocompatible material with an elasticity that is several fold PTFE's inherent elasticity by, e.g. weaving or knitting a PTFE fiber. Lace knitting generally produces the most flexible fabric, since it has large holes that can deform in shape; by contrast, cable knitting generally produces the least flexible fabric, since the stitches are crossed under tension, which inhibits deformation. Knitted fabrics that do not deform much are called stable knits. For comparison, woven fabrics typically deform only along their bias direction - i.e., at 45° to the warp and weft directions - and only by a small amount; however, a woven fabric made with an elastic material may deform more than a stable knit. Thus, the skilled person is well aware on how to produce sheet or tubular structures that show the desired elasticity in the desired directions, in particular along the length and width of the sheet or in radial direction and/or along the longitudinal axis of the tubular structure. The elasticity allows, e.g. tubular structures of a given diameter to be tightly fitted to implants having different diameters and shapes.

The term "bioresorbable" refers to material that is dissolved or degraded when contact with tissue and/or fluids in the body of a patient by e.g. enzymatic or chemical means. Preferred bioresorbable materials comprise silk, cellulose and homo- or copolymers, or a blend thereof wherein the homo- or copolymers comprise monomers selected from the group consisting of glycolic acid, lactic acid, caprolactone, trimethylene carbonate, paradioxanone and 1,5 dioxepan-2-one and optionally a plasticizer selected from the group including ethyl, butyl and hexyl esters of acetylated or non-acetylated citric acid, ethyl terminated oligomers of lactic acid. In this context, degradation is defined as a chemical change in said material and preferably the degradation process is accompanied by cleavage of chemical bonds in the material and a lowering of its molar mass.

As used herein, a "hydrogel" is a network of polymer chains that are water-insoluble, sometimes found as a colloidal gel in which water is the dispersion medium. Hydrogels are superabsorbent natural or synthetic polymers and contain preferably over 50%, over 60%, over 70%, over 80%, over 90% and most preferably over 99% water. Hydrogels possess also an elasticity very similar to natural tissue, due to their significant water content.

As used herein, "biocompatible" or "physiologically acceptable" materials and solutions are materials and solutions that do not elicit an undesirable detrimental, toxic or allergic response in vivo, e.g. in the patient.

As used herein, a "polymer" is a compound that includes two or more, preferably 10, 20, 30, 50, 60, 70, 80, 90, 100, 200, 300, 400, 500, 600, 700, 800, 900, 1000 or more covalently linked monomers. A polymer can be made from one type of monomer or more than one type of monomer. The term "polymer" therefore encompasses copolymers, including blockcopolymers in which different types of monomer are grouped separately within the overall polymer. A polymer can be linear or branched.

As used herein, a "prodrug" is any compound that undergoes biotransformation before exhibiting its pharmacological effects. Prodrugs can thus be viewed as biologically active moieties (such as drugs) containing specialized non-toxic protective groups used in a transient manner to alter or to eliminate undesirable properties in the parent molecule.

As used herein, the term "peptide" includes one or more peptides, peptide derivatives, or combinations thereof. Thus, the terms "peptide", "peptides", and "derivatives of peptides" are used interchangeably throughout. "Peptide" refers to both naturally occurring peptides and synthesized peptides, including naturally or non-naturally occurring amino acids. Peptide derivatives are created by chemically modifying a side chain or a free amino or carboxy-terminus of a natural or non-naturally occurring amino acid. This chemical modification includes the addition of further chemical moieties as well as the modification of functional groups in side chains of the amino acids. A peptide is a polymer of between 3 and 50 amino acids, preferably having more than 3, 5, 10, 15, 20, 30, 40 amino acids. The term "protein" includes one or more proteins, protein derivatives, or combinations thereof and is differentiated from the term "peptide" in that it refers to polymers comprising amino acids chains of more than 50 amino acids.

As used herein, "growth factors" are chemicals that regulate cellular metabolic processes, including but not limited to differentiation, proliferation, synthesis of various cellular products, and other metabolic activities. Growth factors may include several families of chemicals, including but not limited to cytokines, eicosanoids, and differentiation factors.

The implants of the prior art, which are coated with drugs suffer from an inherent inflexibility, since the process used for coating of the materials like Ti is usually complex and can not be carried out at the point-of care. Accordingly, it is not possible to vary the amount, type or location of the drug in response to the needs of the patient as determined during surgery. The present invention provides a solution to this and other problems in the prior art by providing a sheet or tubular structure, which consists of biocompatible material is elastic and comprises at least one biologically active substance in at least one region. It is preferred that the sheet or tubular structure comprises one, two, three, four, five or more further biologically active substances in the same of different region(s). In one embodiment the sheet or tubular structure may comprise two, three, four, five, six, seven eight or more regions. Preferably, the one, two, three, four, five, six, seven, eight or more region(s) do not extend over the entire outer and/or inner surface or the sheet or tubular structure but only cover between 5% and 95% of the outer and/or inner surface, preferably less then 90%, 80%, 70%, 60%, 50%, 40%, 30%, 20%, 10% of the outer surface and/or inner surface.

The sheet or tubular structure of the invention can also be used as such, e.g. without a biologically active substance, for example, to place it as a supporting structure close to an organ or regenerating tissue that needs help with its endogenous healing process (e.g. like a muscle tissue around a fracture). Thus, in a further aspect, the invention provides a sheet or tubular structure which consists of biocompatible material and is elastic. Preferably the sheet or tubular structure of the invention, irrespective whether it does or does not comprise at least one biologically active substance in at least one region, is sterile. Otherwise it preferably has the preferred characteristics and dimensions outlined above and below in more detail, in particular length, width or diameter, thickness and elasticity.

It is frequently desired to control the release kinetic of the biologically active substance from the sheet or tubular structure *in vivo,* i.e. in the patient. Thus, in a preferred embodiment of the invention the sheet or tubular structure of the invention further comprises a biologically active substance release-modifying agent. A suitable biologically active substance release-modifying agent can be chosen by the skilled person based on the guidance provided in this specification and his general knowledge based on, e.g. the type of biological active substance to be released, the planed implantation site, e.g. direct contact with the blood circulation or not, and/or whether the release rate should be increased or decreased. Accordingly, in a second aspect the invention provides a sheet or tubular structure, which consists of a biocompatible material, is elastic and comprises at least one biologically active substance release-modifying agent in at least one region. Depending on the biologically active substance the release of which is modified the region may comprise one or more further biologically active substance release-modifying agent. In one embodiment the sheet or tubular structure may comprise two, three, four, five, six, seven eight or more regions. Preferably, the one, two, three, four, five, six, seven, eight or more region(s) do not extend over the entire outer and/or inner surface or the sheet or tubular structure but only cover between 5% and 95% of the outer and/or inner surface, preferably less then 90%, 80%, 70%, 60%, 50%, 40%, 30%, 20%, 10% of the outer surface and/or inner surface. The region(s) to which the biologically active substance release-modifying agent is applied are preferably the regions to which the biologically active substance may be applied in a later step.

In a preferred embodiment the sheet or tubular structure comprises both at least one biologically active substance in at least one region and at least one biologically active substance release-modifying agent in at least one region. It is preferred that the biologically active substance and the biologically active substance release-modifying agent are comprised in the same region. Since different biologically active substances may require different biologically active substance release-modifying agents to modify, e.g. increase or decrease the release of the biologically active substance, it is envisioned that the sheet or tubular structure comprises two or more different biologically active substances in two or more different regions, wherein each region further comprises the appropriate biologically active substance release-modifying agent.

The tubular structure is particularly preferred since it allows draping the implant more easily by slipping the elastic tubular structure over at least part of an implant, preferably over the entire length of an implant. The tubular structure obviates the need to connect two sides of a sheet to cover the full circumference of an implant and, thus, requires less manipulation in these applications then a sheet structure. The term "tubular structure" refers to a structure having in at least one section a continuous inward facing surface and a continuous outward facing surface. Preferably the tubular structure has a circular or oval cross-section, if not extended by force. In a particularly preferred embodiment the tubular structure has a cylindrical shape. In a preferred embodiment, the tubular structure is sealed at one end. In this context "sealed" does not necessarily mean a fluid tight seal but rather indicates that one side of the tubular structure is closed. Such a tubular structure, which is closed at one side, may be slipped over an implant until the implant reaches the closed end, which will allow, e.g. a better positioning of the one or more regions in the tubular structure comprising the biologically active substance and/or the biologically active substance release-modifying agent and/or a tighter fit between the tubular structure and the implant.

It is also preferred that the biocompatible material is capable of locally releasing said biologically active substance under physiological conditions. Thus, the biocompatible material is preferably chosen in such that it does not bind irreversibly to the biologically active substance. The physiological conditions are those typically found at the site of implantation, e.g. 37°C and approx. pH 7.4.

In a further preferred embodiment, the biocompatible material of the invention comprises, essentially consists, or consists of (i) a fabric comprising, essentially consisting, consisting of weaves, knits, braids, filament windings, spun windings, a felt or combinations thereof and/or (ii) a membrane or a foil. Preferably, the fabric may be either woven or non-woven. Furthermore, said fabric preferably comprises, essentially consists or consists of fibers of natural polymers, fibers of artificial polymers, fibers of mixed natural and artificial polymers and mixtures thereof.

The fabric preferably has a thickness of between 0.03 mm and 1 mm, preferably the fabric has a thickness of less than 0.9 mm, 0.8 mm, 0.7 mm, 0.6 mm, 0.5 mm, 0.4 mm, 0.3 mm, 0.2 mm, 0.1 mm, 0.09 mm, 0.08 mm, 0.07 mm, 0.06 mm, 0.05 mm or less than 0.04 mm. The preferred thickness of the fiber making up the fabric is between 0.01 mm and 1 mm, preferably the fiber has a thickness of less than 0.5 mm, 0.4 mm, 0.3 mm, 0.2 mm, 0.1 mm, 0.09 mm, 0.08 mm, 0.07 mm, 0.06 mm, 0.05 or less than 0.04 mm. In a preferred embodiment the fiber can be electro spun or nano spun. The preferred thickness of the membrane or foil is between 0.03 mm and 1 mm, preferably the fabric has a thickness of less than 0.9 mm, 0.8 mm, 0.7 mm, 0.6 mm, 0.5 mm, 0.4 mm, 0.3 mm, 0.2 mm, 0.1 mm, 0.09 mm, 0.08 mm, 0.07 mm, 0.06 mm, 0.05 mm or less than 0.04 mm.

In a preferred embodiment, the natural polymers are selected from the group consisting of silk, cotton, cellulose, agarose, methylcellulose, hyluronan and derivatives thereof and the artificial polymers are selected from the group consisting of poly(ethylene oxide) (PEO), polytetrafluoroethylene (PTFE), polyethylene terephthalate (PET), polyvinyl alcohol, acrylate polymers, silicone and derivatives thereof. Preferred homo- or copolymers comprise monomers selected from the group consisting of glycolic acid, lactic acid, caprolactone, trimethylene carbonate, paradioxanone and 1,5 dioxepan-2-one and optionally a plasticizer selected from the group including ethyl, butyl and hexyl esters of acetylated or non-acetylated citric acid, ethyl terminated oligomers of lactic acid.

In a further embodiment the biocompatible material of the invention comprises, essentially consists or consists of a hydrogel. The hydrogel is preferably selected from the group consisting of PHEMA, a hyaluronic acid hydrogel, a poly(ethylene glycol) hydrogel, a dextran-based hydrogel, a poly-acrylamide-based hydrogel and a poly(acrylic)-based hydrogel.

Preferably, the sheet or tubular structure of the invention is tearproof. Thus, preferably, the biocompatible material comprises, essentially consists or consists of polymers and/or threads of high tensile strength exceeding preferably a tensile strength of 0.3, 0.5, 0.7, 1.0, 1.3, 1.5 GPa or more. Preferably, such polymers and/or threads of high tensile strength are embedded and/or woven into a biocompatible material which makes up the sheet or tubular structure of the present invention. For example, single stronger threads which preferably are thicker than the remaining threads can be included to increase the tearproof properties.

To hold a therapeutically effective amount of the biologically active substance it is desirable that the biologically active substance is not solely located on the surface of the biocompatible material but also penetrates all or part of the biocompatible material. To this end the surface of the fibers or the felts may be porous to provide better liquid retaining properties. In a further preferred embodiment, the biocompatible material is capable of absorbing and/or adsorbing the biologically active substance.

In preferred applications the sheet or tubular structure of the present invention will provide the biologically active substance for a certain period of time, e.g. during in growth of bone tissue. Thus, after a given period the presence of the sheet or tubular structure is no longer required and, thus, it is preferred that the biocompatible material of the invention is bioresorbable. A large number of bioresorbable polymers having different degradation times are known to the skilled person, which can be chosen according to the desired disintegration time and can be used in the present invention. In one embodiments of the present invention the biologically active substance is embedded in the bioresorbable material and is released upon degradation of the sheet or tubular structure. In this embodiment typically the entire sheet or tubular structure will comprise the biologically active substance. Accordingly, it is preferred that biologically active substances are embedded that are usable for the majority of patients and which are relatively cheap, e.g. antibiotics or antimycotics. It is then possible to add one or more further biologically active substance, e.g. a more expensive one, to at least one region of the sheet or tubular structure. This may lead to a sheet or tubular structure that comprises a first biologically active substance homogenously distributed and one, two, three or more further biologically active substances only a part of the sheet or structure.

In a preferred embodiment of the tubular structure has a diameter of between 0.2 and 5 cm, preferably a diameter which is larger than 0.2, 0.3, 0.4, 0.5, 0.6, 0.7, 0.8, 0.9, 1.0, 1.5, 2.0, 2.5, 3.0, 3.5, 4.0, or 4.5 cm. Due to the flexibility of the material these diameters will be suitable to drape any implant or existing bone structure. The sheet may have various widths, it is however, preferred that the sheet has a width between 0.5 cm and 15 cm, in particular 1.0, 1.5, 2.0, 2.5, 3.0, 4.0, 5.0, 6.0, 7.0, 8.0, 9.0, or 10 cm.

Preferably, the sheet or tubular structure is provided in long sizes, which may have a length between 1 m and 500 m or more, in particular 1, 2, 3, 4, 5, 6, 7, 8, 9, 10, 11, 12, 15, 20, 25, 50, 100, 150, 200, 250, 300, 350, 400, 450 or 500 m, or are within a range defined by any two of these values. The tubular structure may be flattened during storage and may only expose its inner surface once it is removed from, e.g. a role holding the tubular structure. It is preferred that a piece of suitable length will be cut by the practitioner at the point-of-care depending on the requirements of the patient. Preferably, the sheet or tubular structure is protected from the environment by a suitable dispenser, which preferably preserves the sterility of the tube or tubular structure. Thus, a further aspect of the invention is a dispenser comprising the sheet or tubular structure of the invention, preferably on a role. In some embodiments the sheet or tubular structure is provided in sheets or tubular structures pre-cut to typically required lengths. This pre-cut sheets or tubes may be individually packed. Preferred lengths are between 1 cm to 100 cm, in particular 1 cm, 2 cm, 3 cm, 4 cm, 5 cm, 6 cm, 7 cm, 8 cm, 9 cm, 10 cm, 11 cm, 12 cm, 13 cm, 14 cm, 15 cm, 16 cm, 17 cm, 18 cm, 19 cm, 20 cm, 25 cm, 30 cm, 35 cm, 40 cm, 45 cm, 50 cm, 55 cm, 60 cm, 65 cm, 70 cm, 75 cm, 80 cm, 85 cm, 90 cm, 95 cm or 100 cm or are within a range defined by any two of these values.

Preferably, the sheet or tubular structure is sterile. Sterilization may be achieved with any art known method including, e.g. γ-irradiation, heat treatment or chemical treatment. The most suitable sterilization method is determined by the biocompatible material used. Typically, the sterilization is carried out prior to the application of the biologically active substance(s) and/or the biologically active substance release-modifying agent(s).

In a preferred embodiment of the sheets or tubular structures of the present invention the biologically active substance is a drug or a prodrug, preferably selected from the group consisting of an adrenocorticostatic, a β-adrenolytic, an androgen or antiandrogen, an antianemic, an antiparasitic, an anabolic, an anaesthetic or analgesic, an analeptic, an antiallergic, an antiarrhythmic, an antiarterosclerotic, an antibiotic, an antidiabetic, an antifibrinolytic, an anticonvulsive, an angiogenesis inhibitor, an anticholinergic, an enzyme, a coenzyme or a corresponding inhibitor, an antihistaminic, an antihypertonic, an antihypotonic, an anticoagulant, an antimycotic, an antiinfective, an antihemorrhagic, a beta-receptor and calcium channel antagonist, an antimyasthenic, an antiphlogistic, an antipyretic, an antirheumatic, an antiseptic, a cardiotonic, a chemotherapeutic, a coronary dilatator, a cytostatic, a glucocorticoid, a haemostatic, an immunoglobuline or its fragment, a chemokine, a cytokine, a prodrug of a cytokines, a mitogen, a physiological or pharmacological inhibitor of mitogens, a cell differentiation factor, a cytotoxic agent and prodrugs thereof, a hormone, an immunosuppressant, an immunostimulant, a mineralcorticoid, a morphine-antagonist, a muscle relaxant, a narcotic, a vector, a peptide, a (para)-sympaticomimetic or (para)-sympathicolytic, a protein, a cell, a psoriasis/neurodermitis drug, a selective estrogen receptor modulator (SERM), a sedating agent, a spasmolytic, a substance that inhibits the resorption of bone, a vasoconstrictor or vasodilatator, a virustatic, a wound-healing substance, and combinations thereof.

In a preferred embodiment, the biologically active substance of the invention is utilized in form of a prodrug.

In the following, especially preferred examples of biologically active substances which can be used in the invention are indicated. These examples are not to be construed as limiting. As will be understood by the skilled artisan, some especially preferred substances exhibit more than one therapeutic effect and, thus, some biologically active substances are indicated in several classes of biologically active substances.

A preferred adrenocorticostatic is metyrapon. Preferred examples of a β-adrenolytic include labetalol, atenolol, toliprolol and propyl-3-acetyl-4-[2-hydroxy-3-isopropylamino) propoxy] carbanylate hydrochloride. Preferred examples of an androgen include Dehydroepiandrosterone (DHEA), Androstenedione, Androstenediol, Androsterone and Dihydrotestosterone (DHT). Preferred examples of an antiandrogen include Spironolactone, Cyproterone acetate, Flutamide, Ketoconazole and Finasteride. Preferred examples of an antianemic are ferrous sulfate, ferrous gluconate, vitamin C, folic acid, vitamin b-12, recombinant erythropoietin and epoetin alfa. Preferred examples of an antiparasitic include mebendazole (for most nematode infections), pyrantel pamoate, thiabendazole (for roundworm infections), diethycarbazine (for treatment of Lymphatic filariasis), niclosamide, praziquantel, praziquantel, rifampin, amphotericin B, antiprotozoals and melarsoprol. Preferred examples of an anabolic include growth hormone IGF1, insulin, testosterone, estrogen, orexin, hypocretin and melatonin. Preferred examples of an anaesthetic include procaine, amethocaine, cocaine, lidocaine, prilocaine, bupivicaine, levobupivacaine, ropivacaine and dibucaine. Preferred examples of analgesic include rofecoxib, celecoxib, codeine, oxycodone, hydrocodone, diamorphine, pethidine, ibuprofen and diclofenac. Preferred examples of an analeptic include bicuculline, substituted 1,3-diamino-2-propanols and nikethamide. Preferred examples of an antiallergic include monoclonal anti-IgE antibodies, antihistamines, cortisone, dexamethasone, hydrocortisone, epinephrine (adrenaline), theophylline, cromolyn sodium, montelukast (Singulair) and zafirlukast (Accolate). Preferred examples of an antiarrhythmic include procainamide, quinidine, quinidine, lidocaine, mexiletine, tocainide, phenytoin, encainide, flecainide, moricizine, propafenone, esmolol, propranolol, metoprolol, amiodarone, azimilide, bretylium, clofilium, dofetilide, tedisamil, ibutilide, sematilide, sotalol, verapamil, diltiazem, digoxin and adenosine. Preferred examples of an antiarterosclerotic include rosuvastatin, niacin, ezetimibe, atorvastatin, cerivastatin, fluvastatin, lovastatin, mevastatin, pitavastatin, pravastatin, rosuvastatin, simvastatin, simvastatin, lovastatin and atorvastatin. Preferred examples of an antidiabetic include insulin, sulfonylureas (such as acetohexamide, chlorpropamide), metformin meglitinide (such as nateglinide and repaglinide) and thiazolidinedione (such as pioglitazone and rosiglitazone). Alpha-glucosidase inhibitors. Preferred examples of an antifibrinolytic include aminocaproic acid (ε-aminocaproic acid) and tranexamic acid. Preferred examples of an anticonvulsive include paraldehyde, Phenobarbital, methylphenobarbital, metharbital and barbexaclone, and especially preferred are valproate and carbamazepine. Preferred examples of an angiogenesis inhibitor include bevacizumab and resveratrol. Preferred examples of an anticholinergic include dicyclomine and homatropine methylbromide. Preferred examples of an antihistaminic include mepyramine (pyrilamine), antazoline, diphenhydramine, carbinoxamine, doxylamine, clemastine, dimenhydrinate, pheniramine, chlorphenamine (chlorpheniramine), dexchlorpheniramine, brompheniramine, triprolidine, cyclizine, chlorcyclizine, hydroxyzine, meclizine, promethazine, alimemazine, cyproheptadine, azatadine and ketotifen. Preferred examples of an antihypertensive include 4-phenyl-1-piperazinyl, -piperidinyl and- tetrahydropyridyl derivatives, captopril, enalapril, fosinopril (monopril), lisinopril (zestril), quinapril, ramipril (altace), telmisartan (micardis, pritor), irbesartan (avapro), losartan (cozaar), valsartan (diovan), candesartan (atacand), doxazosin, prazosin, terazosin, atenolol, labetalol, metoprolol (lopressor, toprol-xl), propranolol, nifedipine (adalat®), amlodipine (norvasc), diltiazem, verapamil, aliskiren (tekturna), bendroflumethiazide, chlortalidone and hydrochlorothiazide (also called hctz). Preferred examples of an antihypotensive include dopamine, etilefrin and norepinephrine. Preferred examples of an anticoagulant include warfarin (coumadin), acenocoumarol, phenprocoumon, phenindione, heparin and low molecular weight heparin.

Preferred examples of an antimycotic include natamycin, rimocidin, filipin, nystatin, amphotericin b, miconazole, ketoconazole, lotrimin, lotrimin af, econazole, bifonazole, butoconazole, fenticonazole, isoconazole, oxiconazole, sertaconazole, sulconazole, tioconazole, fluconazole, itraconazole, isavuconazole, ravuconazole, posaconazole, voriconazole and terconazole.

Preferred examples of an antihemorrhagic include vitamin K, HSFand erinacin.

Beta-adrenergic blockers can reduce the risk of fracture partly by increasing bone mineral. Thus, preferred are also beta-receptor antagonists including oxprenolol and pindolol, labetalol and carvedilol, alprenolol, carteolol, levobunolol, mepindolol, metipranolol, nadolol, oxprenolol, penbutolol, pindolol, propranolol, sotalol and timolol, Acebutolol, Atenolol, Betaxolol, Bisoprolol, esmolol, Metoprolol, Nebivolol, Carvedilol, Celiprolol, Labetalol, and Butaxamine.

Preferred examples of a calcium channel antagonist are amlodipine (norvasc), felodipine (plendil), nicardipine (cardene, carden sr), nifedipine (procardia, adalat), nimodipine (nimotop), nisoldipine (sular), nitrendipine (cardif, nitrepin), lacidipine (motens), lercanidipine (zanidip), verapamil (calan, isoptin), gallopamil (d600), and diltiazem. Preferred examples of an antimyasthenic include Caspian Tamin, Guanidine, and Pyridostigmine. Preferred examples of an antiphlogistic are Aquathenol, naproxen, Opyrin and ibuprofene. Preferred examples of an antipyretic include aspirin and acetaminophen. Preferred examples of an antirheumatic include adalimumab, azathioprine, chloroquine, hydroxychloroquine (antimalarials), cyclosporine (cyclosporin a), d-penicillamine, etanercept, gold salts (sodium aurothiomalate, auranofin), infliximab, leflunomide, methotrexate (mtx), minocycline (a tetracycline antibiotic) and sulfasalazine (ssz).

Preferred examples of an antiseptic include sodium benzoate, benzalkonium chloride (BAC), cetyl trimethylammonium bromide (CTMB), cetylpyridinium chloride (Cetrim), cetylpyridinium chloride (CPC), benzethonium chloride (BZT), chlorhexidine, octenidine, boric acid, Chlorhexidine Gluconate, hydrogen peroxide, iodine, bis-(dihydropyridinyl)-decane derivatives, 2-phenoxyethanol, phenol, thymol, hexachlorophene, triclosan, sodium 3,5-dibromo-4-hydroxybenzenesulfonate (Dibromol), and sodium hypochlorite. Preferred examples of a cardiotonic include SCH00013, amrinone, AR-L 57, MCI-154, milrinone and neuquinon.

After removal of a bone tumor, frequently implants can be used to fill the resulting bone cavity. Dependent on the individual patient, it may be advantageous to add a chemotherapeutic substance directly to the implant according to the invention by means of the biocompatible material comprising at least one biologically active substance in at least one region. In particular, preferred examples of a chemotherapeutic comprise imatinib mesylate, cisplatin, carboplatin, azathioprine, vinca alkaloids, vincristine, vinblastine, vinorelbine, vindesine, paclitaxel, irinotecan and topotecan.

Preferred examples of a coronary dilatator and/or vasodilatator include glyceryl trinitrate, adenosine, amyl nitrite, 1-arginine, atrial natriuretic peptide (anp), bradykinin, ethanol, endothelium-derived hyperpolarizing factor (edhf), histamine, complement proteins c3a, c4a and c5a, niacin, nitric oxide, isosorbide mononitrate, isosorbide dinitrate, pentaerythritol tetranitrate (petn), sodium nitroprusside, sildenafil, tadalafil, vardenafil, platelet activating factor (paf), prostacyclin (pgi2), tetrahydrocannabinol (thc), theobromine, and papaverine.

Preferred examples of a cytostatic include azathioprine, mercaptopurine, vincristine, vinblastine, vinorelbine, vindesine, podophyllotoxin, docetaxel, irinotecan, topotecan, amsacrine, etoposide, etoposide phosphate, and teniposide.

Preferred examples of a glucocorticoid include hydrocortisone (cortisol), cortisone acetate, prednisone, prednisolone, methylprednisolone, dexamethasone, betamethasone, triamcinolone, beclometasone, fludrocortisone acetate, deoxycorticosterone acetate (doca), and aldosterone.

Preferred examples of a haemostatic include Naphthionin, Desmopressin and Dencichine (β-N-oxalyl-1-α,β-diaminopropionic acid).

The biology and means of production of immunoglobulines and antibodies are well known in the art (see for example Janeway CA, Jr et al (2001). Immunobiology., 5th ed., Garland Publishin). Thus, the biologically active substance may also include immunoglobulins and antibodies directed against a target in the patient, e.g. protein that elicits an undesirable activity.

Cytokine are proteins secreted by cells. Preferred examples of a cytokine is any chemokine such as CCL1 Scyal, CCL2, CCL3, CCL4, CCL5, CCL6, CCL7, CCL8, CCL9/CCL10, CCL11, CCL12, CCL13, CCL14, CCL15, CCL16, CCL17, CCL18, CCL19, CCL20, CCL21, CCL22, CCL23, CCL24, CCL25, CCL26, CCL27, CCL28, CXCL1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCL17, CX3C and CX3CL1. Further cytokines are e.g. familiy members of interleukines such as IL-4, IL-10, IL-13, IL-2, IL-10, IL-17, IL-1 (including IL-1 and IL-18). Further preferred cytokines are TGF-β, IFN-γ, erythropoietin (EPO) and thrombopoietin (THPO).

Preferred examples of a mitogen include phytohaemagglutinin (PHA), concanavalin A (conA), lipopolysaccharide (LPS) and pokeweed mitogen (PWM).

Preferred examples of a physiological or pharmacological inhibitor of mitogens are Rapamycin, AZD6244, U0126, PD 098059 and ethanol.

A preferred cell differentiation factor can be e.g. any growth differentiation factors (GDFs) such as GDF1, GDF2 (also known as BMP9), GDF3 (which occurs in ossifying bone), GDF5, GDF6, GDF8, GDF9, GDF11, GDF 15 and GDF 10. GDF10 also closely related to BMP3 that plays a role in head formation and in skeletal morphogenesis. It is also known as BMP-3b. Also preferred growth factors are Bone Morphogenetic Proteins (BMPs). These proteins are known for their ability to induce the formation of bone and cartilage and are, thus, especially advantageous biologically active substances which can be used with the biocompatible material accrording to the invention to enhance implants and to improve the healing process. Preferred bone morphogenetic proteins comprise BMP1, BMP2, BMP3, BMP4, BMP5, BMP6, BMP7, BMP8a, BMP8b, BMP10 and BMP15.

Preferred examples of a cytotoxic agents include platinum coordination complexes, ethyleneimenes, methylmelamines, trazines, vinca alkaloids, pyrimidine analogs, purine analogs, alkylsulfonates, folic acid analogs, anthracendiones, substituted urea, methylhydrazin derivatives, in particular acediasulfone, aclarubicine, ambazone, aminoglutethimide, L-asparaginase, azathioprine, bleomycin, busulfan, calcium folinate, carboplatin, carpecitabine, carmustine, celecoxib, chlorambucil, cis-platin, cladribine, cyclophosphamide, cytarabine, dacarbazine, dactinomycin dapsone, daunorubicin, dibrompropamidine, diethylstilbestrole, docetaxel, doxorubicin, enediynes, epirubicin, epothilone B, epothilone D, estramucin phosphate, estrogen, ethinylestradiole, etoposide, flavopiridol, floxuridine, fludarabine, fluorouracil, fluoxymesterone, flutamide fosfestrol, furazolidone, gemcitabine, gonadotropin releasing hormone analog, hexamethylmelamine, hydroxycarbamide, hydroxymethylnitrofurantoin, hydroxyprogesteronecaproat, hydroxyurea, idarubicin, idoxuridine, ifosfamide, interferon α, irinotecan, leuprolide, lomustine, lurtotecan, mafenide sulfate olamide, mechlorethamine, medroxyprogesterone acetate, megastrolacetate, melphalan, mepacrine, mercaptopurine, methotrexate, metronidazole, mitomycin C, mitopodozide, mitotane, mitoxantrone, mithramycin, nalidixic acid, nifuratel, nifuroxazide, nifuralazine, nifurtimox, nimustine, ninorazole, nitrofurantoin, nitrogen mustards, oleomucin, oxolinic acid, pentamidine, pentostatin, phenazopyridine, phthalylsulfathiazole, pipobroman, prednimustine, prednisone, preussin, procarbazine, pyrimethamine, raltitrexed, rapamycin, rofecoxib, rosiglitazone, salazosulfapyridine, scriflavinium chloride, semustine streptozocine, sulfacarbamide, sulfacetamide, sulfachlopyridazine, sulfadiazine, sulfadicramide, sulfadimethoxine, sulfaethidole, sulfafurazole, sulfaguanidine, sulfaguanole, sulfamethizole, sulfamethoxazole, co-trimoxazole, sulfamethoxydiazine, sulfamethoxypyridazine, sulfamoxole, sulfanilamide, sulfaperin, sulfaphenazole, sulfathiazole, sulfisomidine, staurosporin, tamoxifen, taxol, teniposide, tertiposide, testolactone, testosteronpropionate, thioguanine, thiotepa, tinidazole, topotecan, triaziquone, treosulfan, trimethoprim, trofosfamide, UCN-01, vinblastine, vincristine, vindesine, vinblastine, vinorelbine, and zorubicin, or their respective derivatives or analogs thereof. Assays to measure cytotoxicity are known in the art and comprise e.g., the MTT assay, Trypan blue (TB) assay, Sulforhodamine B (SRB) assay, WST assay and clonogenic assay.

Any hormone can be used in the invention. Preferred examples of a hormone include Calcitonin, Parathyroid hormone, Calcitriol (Vitamin D3), Epinephrine and Norepinephrine.

Preferred examples of an immunosuppressant include cyclosporine, Tacrolimus, Sirolimus, IFN-β, infliximab (Remicade®), etanercept (Enbrel®), or adalimumab (Humira®), Mycophenolic acid and FTY720.

Preferred examples of an immunostimulant include granulocyte macrophage colony-stimulating factor and Macrokines.

Preferred examples of a mineralcorticoid include Aldosterone, Cortisone, Hydrocortisone/cortisol, Desoxycortone, Alclometasone, Amcinonide, Beclometasone, Betamethasone, Budesonide, Ciclesonide, Clobetasol, Clobetasone, Clocortolone, Cloprednol, Cortivazol, Deflazacort, Deoxycorticosterone, Desonide, Desoximetasone, Dexamethasone, Diflorasone, Diflucortolone, Difluprednate, Fluclorolone, Fludrocortisone, Fludroxycortide, Flumetasone, Flunisolide, Fluocinolone acetonide, Fluocinonide, Fluocortin, Fluocortolone, Fluorometholone, Fluperolone, Fluprednidene, Fluticasone, Formocortal, Halcinonide, Halometasone, Hydrocortisone aceponate, Hydrocortisone buteprate, Hydrocortisone butyrate, Loteprednol, Medrysone, Meprednisone, Methylprednisolone,Methylprednisolone aceponate, Mometasone furoate, Paramethasone, Prednicarbate, Prednisone, Prednisolone, Prednylidene, Rimexolone, Tixocortol, Triamcinolone and Ulobetasol.

Preferred examples of a morphine-antagonist include naloxone, naltrexone, ketamine and dextromethorphan.

Preferred examples of a muscle relaxant include Succinylcholine, Mivacurium, Rapacuronium, Atracurium, Cisatracurium, Vecuronium, Rocuronium, Pancuronium, Metocurine, d-Tubocurarine, Gallamine, Alcuronium, Doxacurium and Pipecuronium.

Preferred examples of a narcotic are opium, opium derivatives, and their semi-synthetic or fully synthetic substitutes as well as cocaine.

The term "vector" as used herein includes any vector known to the skilled person including plasmid vectors, cosmid vectors, phage vectors such as lambda phage, viral vectors such as adenoviral, retroviral, adenovirus-associated-virus (AAV) or baculoviral vectors, or artificial chromosome vectors such as bacterial artificial chromosomes (BAC), yeast artificial chromosomes (YAC), or P 1 artificial chromosomes (PAC). Said vectors include expression as well as cloning vectors. The term "expression vector" refers to a vector that contains a desired coding sequence and appropriate DNA sequences necessary for the expression of the operably linked coding sequence and is capable of inducing protein expression in a particular host organism (e.g., bacteria, yeast, plant, insect, or mammal) or in *in vitro* expression systems. Expression vectors for use in mammalian cells preferably include a promoter located in front of the gene to be expressed, along with any necessary ribosome binding sites, RNA splice sites, polyadenylation site, and/or transcriptional terminator sequences. To obtain stable expression for an extended period of time the expression vector may either be capable of integrating into the host genome or it may contain an origin of replication (ORI). ORIs suitable for stable maintenance of expression vectors are well known in the art and comprise, e.g. for yeast cells CEN and ARS and for mammalian cells ORIs derived from extra-chromosomally replicating viruses, such as Simian Virus 40 (SV40), Polyoma, Adeno, Cytomegalie virus (CMV), vesicular stomatits virus (VSV), or bovine papilloma virs (BPV).

In a preferred embodiment, the vector is a polynucleotide or a nucleotide.

Preferred examples of a nucleotide include adenosine monophosphate, adenosine diphosphate, adenosine triphosphate, guanosine monophosphate, guanosine diphosphate, guanosine triphosphate, thymidine monophosphate, thymidine diphosphate, thymidine triphosphate, uridine monophosphate, uridine diphosphate, uridine triphosphate, cytidine monophosphate, cytidine diphosphate, cytidine triphosphate, deoxyadenosine monophosphate, deoxyadenosine diphosphate, deoxyadenosine triphosphate, deoxyguanosine monophosphate, deoxyguanosine diphosphate, deoxyguanosine triphosphate, deoxythymidine monophosphate, deoxythymidine diphosphate, deoxythymidine triphosphate, deoxyuridine monophosphate, deoxyuridine diphosphate, deoxyuridine triphosphate, deoxycytidine monophosphate, deoxycytidine diphosphate, deoxycytidine triphosphate and derivatives thereof.

Preferred examples of a sympathicomimetica include Terbutaline, Carbaminoylcholine and etilefrin hydrochloride.

Preferred examples of a parasympathomimetics include acetylcholine, bethanechol, carbachol, methacholine, plant alkaloids, nicotine, muscarine, pilocarpine, donepezil, edrophonium, neostigmine, physostigmine, pyridostigmine, rivastigmine, tacrine, echothiophate, isoflurophate, malathion, cisapride and metoclopramide.

Preferred examples of a sympatholytic include Dibenzyline, moxonidine, beta-adrenergic blockers and α2-adrenergic agonists. A preferred example of a parasympatholytic is Homatropine Methylbromide.

Preferred examples of a sedating agent include mirtazapine (Remeron®), trazodone (Desyrel®), amobarbital (Amytal®), pentobarbital (Nembutal®), secobarbital (Seconal®, alprazolam (Xanax®), clonazepam (Klonopin®), diazepam (Valium®), estazolam (Prosom®), flunitrazepam (Rohypnol®), lorazepam (Ativan®), nitrazepam (Mogadon®), oxazepam (Serax®), triazolam (Halcion®), chlorpromazine (Thorazine®, Largactil®), fluphenazine (Prolixin®), haloperidol (Haldol®), loxapine succinate (Loxitane®), Perphenazine (Etrafon®, Trilafon®), prochlorperazine (Compazine®), thiothixene (Navane®), trifluoperazine (Stelazine®, Trifluoperaz®), clozapine (Clozaril®), olanzapine (Zyprexa®), quetiapine (Seroquel®), risperidone (Risperdal®), ziprasidone (Geodon®) (May cause somnolence in some, while causing insomnia in others), Clemastine, Doxylamine, Diphenhydramine, Niaprazine, Pyribenzamine, Ashwagandha extract, Catnip extract, Kava (Piper methysticum) extract, Mandrake extract, Marijuana, Valerian, chloral hydrate (Noctec®), diethyl ether (Ether), ethyl alcohol, gamma-hydroxybutyrate (GHB), methyl trichloride (Chloroform), eszopiclone (Lunesta®), zaleplon (Sonata®), zolpidem (Ambien®), zopiclone (Imovane®, Zimovane®), cyclobenzaprine (Flexeril®), ethchlorvynol (Placidyl®), glutethimide (Doriden®), ketamine (Ketalar®, Ketaset®), methaqualone (Sopor®, Quaalude®), methyprylon (Noludar®) and ramelteon (Rozerem®).

Preferred examples of a spasmolytic include muscle relaxants, narcotics, oxybutynin and dihydrocodeine.

Preferred examples of a cell include a mesenchymal cell, an embryonic stem cell, an osteoblast, an osteoclast, a fibroblast and a platelet.

Preferred examples of a vasoconstrictor include adenosine triphosphate, amphetamines, antihistamines, asymmetric dimethylarginine, catecholamines, cocaine, decongestants, endothelin, ergine, methylphenidate, neuropeptide y, norepinephrine, phenylephrine, pseudoephedrine, stimulants, tetrahydrozoline hydrochloride and thromboxane.

Also preferred are biologically active substances according to the invention which comprise proteins. In a preferred embodiment the protein is an enzymes and preferably said enzyme is selected from the group consisting of: adenosine deaminase, agalsidase, alteplase, anistreplase, ancrod serine protease, asparaginases, Dnase, galactosidase, hyaluronidases, idumonidase, lactase, pancrelipase, papain, platelet activating factor acetylhydrolase (PAF-AH), superoxide dismutase (SOD), streptokinase, sucrase, tilactase, urate oxidase and urokinase.

Preferred examples of a virustatic are interleukins, azidothymidine (AZT), Foscarnet (Foscavir), Ribavarin (1-beta-D-ribofuranosyl-1,2,4-triazole-3-carboxamide), Acyclovir and brivudin.

Preferred examples of a wound-healing substance are thymosin beta 4, angiopoietin-1, TGF-beta, ketanserin, NGF, carnosine, metronidazole, tinidazole and fluconazole.

Especially preferred is a biocompatible material of the invention, wherein the biologically active substance comprises a selective estrogen receptor modulator (SERM) which is selected from the group consisting of clomifene, raloxifene, tamoxifen, toremifene, bazedoxifene, lasofoxifene and ormeloxifene.

In a further preferred embodiment, the biologically active substance according to the invention comprises a substance that inhibits the resorption of bone and which is selected from the group consisting of strontium ranelate, teriparatide and bisphosphonate.

In a further preferred embodiment, the biologically active substance according to the invention comprises a protein which is matrix metalloproteinase (MMP) or bone morphogenetic protein (BMP).

In a further preferred embodiment, the biologically active substance according to the invention comprises an antibiotic which is selected from the group consisting of an aminoglycoside, an ansamycin, a carbacephem, a carbapenem, a cephalosporin, a macrolide, a monobactam, a tetracycline, an oxalidinone, a beta-lactam antibiotic, a quinolone, a sulfonamide, a rifamycin, a glycopeptide antibiotic, a nitrofurantoin, a fusidic acid, or a pharmaceutically acceptable salt thereof. In a more preferred embodiment the antibiotic is selected from the group consisting of gentamycin, kanamycin, netilmicin, penicillin, streptomycin, amikacin, tobramycin, chloramphenicol, erythromycin lincomycin, rifampin, tetracycline, doxycycline, chlortetracycline, minocycline, linezoloid, penicillin, metronidazole, cephalosporin, dicloxacillin, carbapenem, ampicillin, cefepime, ceftazidime, cefotaxime, cefuroxime, cefaclor, cetriaxone, imipenem, meropenem, ciprofloxacin, moxifloxacin, levofloxacin, sulfamethoxazole, vancomycin and derivatives thereof.

As mentioned before, it is preferred that the biologically active substance is released from the biologically biocompatible material of the invention under physiological conditions. This release can be a spontaneous burst release, i.e. the entire biologically active substance or substances are released within a short time, preferably within 10, 20, 30, 40, 50, 60, 120, 240, 460, 800 or 1,000 minutes. Alternatively, the release can be a controlled release, i.e. the substance is released gradually over time, preferably the entire substance is released in the course of one, two, three, four, five, six, seven, eight, nine, ten days or in the course of one, two, three, four, five, six, seven, eight, nine or ten weeks and even more preferably over the course of one, two, three, four, five, six, seven, eight, nine or ten months.

The kinetic of the release of the biologically active substance can be modulated by selecting and including various different release-modifying agents in the biologically biocompatible material of the invention. The release-modifying agent may increase or decrease the release rate of the biological active substance from the sheet or tubular structure, if compared to a sheet or tubular structure comprising the same concentration of the biological active substance alone. The majority of "controlled-release" drug delivery systems are known in the prior art (see, e.g., U.S. Pat. No. 4,145,410 to Sears) which describes a drug release system capable of releasing drugs, i.e. biologically active substances in the human body. Thus a skilled person can utilize such technology comprised in the art to choose a suitable release-modifying agent for the biologically biocompatible material of the invention.

In a preferred aspect of the invention, the biologically active substance release-modifying agent of the invention is selected from serum, preferably autologous serum, cells, preferably platelets, co-solvents, viscosity modifiers, chaotropic agents, polymeric salts, surfactants, polymeric acids, polymeric bases, gels, non-volatile monoprotic or polyprotic organic acid, a non-volatile, mono- or poly-functional base, citric acid and combinations thereof.

Also preferred is a biocompatible material of the invention, wherein the biologically active substance release-modifying agent comprises a reactive group capable of covalently binding at least one biologically active substance. Thus, in embodiments wherein the sheet or tubular structure initially only comprise a biologically active substance release-modifying agent, it is possible to apply the biologically active substance to only some regions of the sheet or tubular structure and still obtain a stable, e.g. covalent, connection between the sheet or tubular structure and the biologically active substance. In a more preferred embodiment, the biologically active substance release-modifying agent acts as a linker, covalently linking and/or binding the biologically active substance to the biocompatible material of the invention. As used herein, "binding" refers to a covalent bond and/or a non-covalent bond, which can be mediated by e.g. a Van der Waals force, a hydrophobic and/or electrostatic interaction.

It is to be understood that the biologically active substance may be linked to the desired biologically active substance release-modifying agent through any number of covalent linkages, including but not limited to amide, ester, ether, isourea, and imine bonds. Further exemplary embodiments of reactive groups and methods of binding a biologically active substance to the biologically active substance release-modifying agent are discussed in more detail below. It is also to be understood that the same methods and/or reactive group or reactive groups can be applied to link the biologically active substance release-modifying agent to the biocompatible material of the invention, *mutatis mutandis.*

In preferred embodiments, the biologically active substance comprises a functional group selected from the group consisting of -COOH, -NH₂, -OH, and -SH. Preferably, a reactive group comprised in the biologically active substance release-modifying agent is capable of forming a covalent bond with this functional group. Reactive groups having a reactivity towards any of the aforementioned functional groups are well known in the art of organic chemistry and peptide chemistry and can be found, for example, in Kim et al. Biomaterials 24:4843 (2003), Baudys et al., Bioconj. Chem. 9:176-183 (1998) and Thoma et al., J. Am. Chem. Soc. 121:5919-5929 (1999).

In a more preferred embodiment, the biologically active substance release-modifying agent further comprises a biocleavable functional group or spacer and/or the bond between the biologically active substance release-modifying agent and the biologically active substance and/or a bond between the biologically active substance release-modifying agent and the biocompatible material is biocleavable. Suitable functional groups and linkers, which may be incorporated into the release-modifying agents and which are cleaved under physiological conditions are known from the prior art and have been describe in, e.g. WO 01/47562 A2, Tsubery et al. (2004) J. Biol Chem 279: 38118-24, Greenwald R.B. et al. (2004) J. Med Chem. 47: 726-734, Greenwald R.B. et al. (2000) J. Med. Chem. 43:475-487, Greenwald R.B. et al. (2003) Bioconjugate 14: 395-403, Lee M.R. et al .(2004) Angew. Chem. 116: 1707-1710, EP 1 625 855 A1, WO 2006/136586 and WO 2006/003014.

In a more preferred embodiment, the region of the sheet or tubular structure of the invention comprising the biologically active substance and/or the biologically active substance release-modifying agent extends over the entire sheet or tubular structure. In this embodiment the practitioner is free to apply the biologically active substance to any region of the sheet or tubular structure without first applying a biologically active substance release-modifying agent.

When the biologically active substance release-modifying agent further comprises a biocleavable spacer and/or bond, it is preferred that upon cleavage *in vivo,* the biologically active substance is released in an unmodified form. A variety of different linking agents or linking groups may be applied to the biologically active substance release-modifying agent for this purpose as described by B.Testa et al. (B. Testa, J. Mayer, Hydrolysis in Drug and Prodrug Metabolism, Wiley-VCH, 2003).

Biodegradable bonds which may be cleaved chemically, preferably hydrolytically, under in vivo conditions include but are not limited to phosphate, phosphonate, carbonate, carbamate, disulfide and ester bonds. There also exists a huge variety of spacers which may be cleaved enzymatically. For example, polypeptides may be used as spacers which comprise target sequences that are cleaved in vivo by specific hydrolytic enzymes. The rate of degradation under in vivo conditions is different for every different type of polypeptide and/or spacer used. In general, the degradation rate is a function of the degradability of the backbone (number of cleavable bonds, dependence of bond cleavage upon autohydrolysis or enzymatic catalysis) and the degree of crosslinking through the spacer and/or bond(s) between the biologically active substance release-modifying agent and the biologically active substance and/or a bond between the biologically active substance release-modifying agent and the biocompatible material. Even though crosslinks do not directly contribute to the degradability of the spacers, the crosslinks can modulate enzyme access to the spacers. Suitable biocleavable materials include but are not limited to carbohydrate-based polymers like dextran, chitosan, hyaluronic acid and derivatives, alginate, xylan, mannan, carrageenan, agarose, cellulose, starch, and hydroxyethyl starch and poly- or oligopeptide based oligomers, polynucleotides, or polymers like synthetic peptide sequences, collagen and gelatin.

A further aspect of the invention is an implant covered at least partially by a sheet or tubular structure of the invention. A preferred implant usable in the present is an orthopedic implant, in particular an elongated plate for coupling severed bone regions, fracture plates, fixator rods, hip implant stems, knee implant stems, K wires, or shoulder plates; an implantable biosensor, an implantable drug infusion tube, an intravitreal drug delivery device, a nerve regeneration conduit, a pacemaker, an implantable electrostimulation lead, in particular electrostimulation lead of a pacemaker, a spinal repair device, a stent, in particular a coronary, peripheral, or GI stent.. Preferably the entire implant is covered by the sheet or tubular structure. If the implant is covered by a tubular structure then it is preferred that one or both ends of the tubular structure are closed, to reduce slip of the tubular structure on the implant. Such closing/sealing is typically effected at the point-of-care and may be effected by appropriate adhesives or, if the biocompatible material is a thermoplastic, by applying heat.

A further aspect of the invention is a method of producing the sheet or tubular structure of the invention comprising the step of contacting a biocompatible material in at least one region with at least one biologically active substance and/or at least one biologically active substance release-modifying agent. As set out before the resultant sheet or tubular structure may comprise one, two, three, four, five, six, seven or more regions comprising the same or different biologically active substances and/or biologically active substance release-modifying agents. Accordingly, the method may comprise several separate application steps or may comprise the application of mixtures of two or more biologically active substances at once. It is preferred that the sheet or tubular structure is contacted with a mixture of a biologically active substance and a biologically active substance release-modifying agent, to ensure that both compounds are present in each region together. In some embodiments the biologically active substance release-modifying agent is biodegradable and a solidifies once applied to the sheet or tubular structure and, thus, will trap any biologically active substance that may be premixed with the release-modifying agent prior to application to the sheet or tubular structure.

The method of the invention has several advantages: the sheet or tubular structure of the invention is customizable according to the needs of the patient. For example, the region of local release, the kinetic of release, the type of biocompatible material and the biologically active substance or mix of biologically active substances can be combined at the point-of-care. This is especially advantageous when biologically active substances once contacted with a biocompatible material have a short half-life.

In a preferred embodiment, in the method of the invention of producing the biocompatible material the biocompatible material is contacted with the biologically active substance release-modifying agent prior to, concomitantly or after contacting the biocompatible material with the biologically active substance.

It is further preferred that the contacting in the method of the invention is effected by dripping at least one biologically active substance onto at least one region of the biocompatible material. This, is preferred if expensive biologically active substances are used, since it provides good control with respect to the amount applied.

In preferred embodiments of the method of the invention, the contacting is carried out in a hospital and/or more preferably in an operating room.

A further aspect of the invention is a method of producing an enhanced implant comprising the steps of the above-stated method of the invention and further comprising the step of at least partially covering an implant with the biocompatible material. Preferably, the step of covering the implant is carried out prior or after the application of the biologically active substance and/or the biologically active substance release-modifying agent to the sheet or tubular structure. It is further preferred that the entire implant is covered.

A further aspect of the invention is a sheet or tubular structure producible by the method of the present invention. A further aspect of the invention is an enhanced implant producible by the method of the invention.

When, e.g. a person has an orthopedic implant surgery, they normally will have to take antibiotics and growth factors either orally or by injection. There are several side effects associated with taking drugs this way which can be very painful for the patient. However, by placing the biologically active substance, e.g. the drug, onto the surface of the implant, the biologically active substances can be delivered right where they are needed and can avoid larger doses and side effect. A customizable/patient specific sheet or tubular structure wrapped around the implant allows the drug to maintain its bioavailability. This also facilitates to deliver the drugs at the physiologically required rate and/or at the desired duration of time. Alternatively, or additionally, the sheet or tubular structure according to the invention can also be implanted as such, i.e. as a drug release form with definable release kinetics, to provide "patient specific drug delivery" and to help patients undergoing hormone therapy, chemotherapy or other treatments requiring periodic medication. Thus, a further aspect of the invention is the use of a sheet or tubular structure according to the invention or the enhanced implant according to the invention, for the treatment of a disease or an ailment selected from the group consisting of a defective, fractured or lost bone, bone-structure or tooth (e.g. for orthopedic treatments), of a disease-induced localized flow constriction (e.g. during bypass operations), of a heart disease (e.g. with improved pacemakers), of chronic or acute pain, of a genetic disorder, of arthritis, of rheumatism of cancer of an inflammation and of an infection. The flow constriction that is treated is preferably a constriction of the flow of a fluid through natural body conduits such as central and peripheral arteries and veins, bile ducts, esophagus, colon, trachea or large bronchi, ureters, and urethra.

As it is preferred that the sheet or tubular structure is individualized for the respective patient at the point of care a further aspect of the invention is an apparatus for producing a sheet or tubular structure according to the invention comprising:
(i) a dispenser device (2) for dispensing one or more sheets or tubular structures (1);
(ii) at least one reservoir (3) capable of containing a biologically active substance and/or a biologically active substance release-modifying agent; and
(iii) a release system (4) capable of releasing a biologically active substance and/or a biologically active substance release-modifying agent from at least one reservoir (3) onto the sheet or tubular structure (1).

The apparatus may comprise one, two, three, four, five, six, seven, eight or more further reservoirs (3) and corresponding release systems (4), depending on the number of biologically active substance(s) and/or a biologically active substance release-modifying agent(s) to be applied to the sheet or tubular structure.

The apparatus of the invention has the advantage that biocompatible materials can be medicated with a drug substance while treating a patient, i.e., on demand, during, for example a surgical procedure. This allows the practitioner to produce an optimal sheet or tubular structure according to the invention comprising a selected combination of biocompatible material, biologically active substance(s) and/or a biologically active substance release-modifying agent(s) which best suit the needs of the individual patent.

The "dispenser device" (2) serves to hold the sheet or tubular structure of the invention. In a simple embodiment the dispenser device may be a rod or cylinder removable connected to supports on one or both ends of the rod or cylinder and onto which is inserted into rolls of the sheet or tubular structures. It may additionally comprise an encasing to preserve the sterility of the sheet or tubular structures.

In a preferred embodiment, the reservoir(s) (3) comprise a biologically active substance release-modifying agent(s) and/or biologically active substance. In that it is particularly preferred that the reservoir(s) (3) of the apparatus of the invention comprise a mixture of one, two, three or more different biologically active substances and/or one, two, three or more different biologically active substance release-modifying agent(s).

In a preferred embodiment, the apparatus according to the invention further comprises a cutting device (5) capable of cutting the sheet or tubular structure (1). Preferably, the cutting device cuts thermally and/or mechanically. Therefore, a cutting device preferably comprises at least one heated wire and/or a blade which can be in form of a saw-tooth-shaped blade. It is also preferred that the cutting device is adjustable, e.g. the angle of the blade, the height of the blade e.t.c. can be changed. The blade is preferably reversibly attached to the apparatus such that it can be replaced, if need be, with a new blade.

In a preferred embodiment of the apparatus, the biocompatible material (1) is tubular and the cutting device is capable of sealing and optionally cutting the tubular biocompatible material.

In a further preferred embodiment, the apparatus according to the invention further comprises a wetting tray (6). The wetting tray preferably allows contacting the entire surface of the sheet or tubular structure (1) with a biologically active substance and/or a biologically active substance release-modifying agent. Typically the tubular structure is flattened out when the biologically active substance(s) and/or a biologically active substance release-modifying agent(s) are applied, which allows to wet both sides of the tubular structure simultaneously.

In a further preferred embodiment, the apparatus according to the invention further comprises a refrigeration unit configured to cool at least one reservoir (3).

It is preferred that the dispenser device (2) of the apparatus according to the invention comprises one or more rolls of the sheet or tubular structure (1).

It is also preferred that the apparatus further comprises a measuring device for measuring the length of the sheet or tubular structure (1) dispensed from the dispenser device (2). The measuring device preferably consists of a scale (8), displaying units of length, and the measuring device is preferably located not farther away than 1, 2, 3, 4, 5, 6, 7, 8, 9, 10, 20, 30, 40, 50 cm from the dispenser.

In a further preferred embodiment, at least one reservoir (3) of the apparatus comprises a controlled release system which permits the release of a specified volume of the biologically active substance and/or a biologically active substance release-modifying agent from the reservoir (3) onto the sheet or tubular structure (1).

The volume of the released biologically active substance(s) and/or a biologically active substance release-modifying agent(s) may be measured and may be translated into an associated cost of the released biologically active substance(s) and/or a biologically active substance release-modifying agent(s). These calculations may take concentration (in cases of applying solutions of biologically active substance(s) and/or a biologically active substance release-modifying agent(s) and/or type of the biologically active substance(s) and/or a biologically active substance release-modifying agent(s) into account. Additionally, the length of the dispensed sheet or tubular structure may be further taken into account. Accordingly, the apparatus of the invention is in a preferred embodiment equipped with a device capable to compute the costs of the dispensed biologically active substance(s) and/or a biologically active substance release-modifying agent and/or sheet or tubular structure of the invention. Additionally, the device may be equipped for inputting costs of individual biologically active substance(s) and/or a biologically active substance release-modifying agent(s) and/or sheets or tubular structures. To allow transferring this information, e.g. into a medical billing and/or accounting system the apparatus of the invention preferably further comprises a communication interface like, e.g. serial port, USB, Ethernet, WIFI etc, which enables communication with a computer system running medical billing and/or accounting software.

In another preferred embodiment, the controlled release system of the apparatus of the invention further comprises an indicator indicating the volume and/or amount and/or costs of the biologically active substance and/or a biologically active substance release-modifying agent which is to be released from the reservoir (3). Preferably, the indicator comprises a scale displaying calibrated units of volume (7), which is attached to or comprised in at least one reservoir (3) which is preferably at least partially translucent.

The measuring device is preferably based on the number of times the dispenser device (2) is pressed. This is may be registered as a number and later be used to calculate the price of drug dispensed.

In preferred embodiments of the apparatus of the invention at least one part of the apparatus is sterile. Preferably the entire apparatus is sterile.

A further aspect of the invention is a method of treating a patient suffering from a disease or an ailment selected from the group consisting of a defective, fractured or lost bone, bone-structure or tooth, of a disease-induced localized flow constriction, of a heart disease, of chronic or acute pain, of a genetic disorder, of arthritis, of rheumatism of cancer of an inflammation and of an infection, wherein the method of treating a patient comprises the steps:
(i) using the apparatus according to the invention to produce a customized sheet or tubular structure according to the invention; wherein the sheet or tubular structure is preferably customized to meet the needs of the patient;
(ii) optionally at least partially covering an implant with the customized sheet or tubular structure; and
(iii) implanting said customized sheet or tubular structure or the implant which is at least partially covered with the customized sheet or tubular structure into the patient.

As used herein, the verbs "comprising" and "consisting" can be used interchangeably.

Various modifications and variations of the invention will be apparent to those skilled in the art without departing from the scope of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in the relevant fields are intended to be covered by the present invention.

The following figures are merely illustrative of the present invention and should not be construed to limit the scope of the invention as indicated by the appended claims in any way.

### BRIEF DESCRIPTION OF THE FIGURES

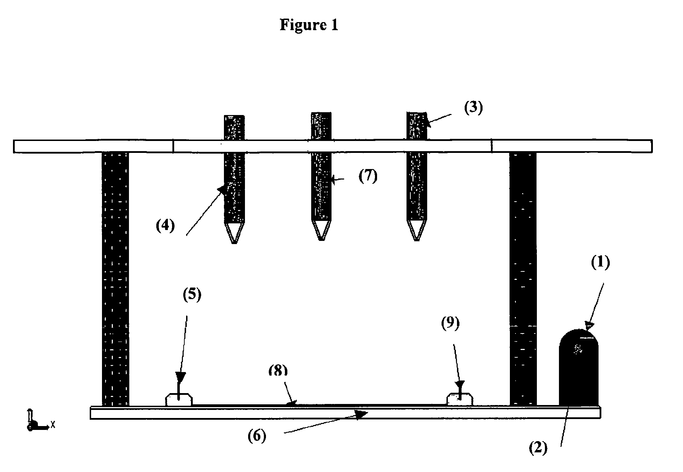

Fig. 1 Example of a preferred apparatus for producing a biocompatible material according to the invention. The a sheet or tubular dispenser device (2), a reservoir (3), an adjustable cutting device (5), a wetting tray (6), a scale of the reservoir (7), a scale (8) to measure lengths of sheet or tubular material and a second blade (9) are indicated. For reasons of clarity, the release system (4) is not shown. However, preferred embodiments of this apparatus may comprise all or some of the features indicated as (1) - (9) in any combination.

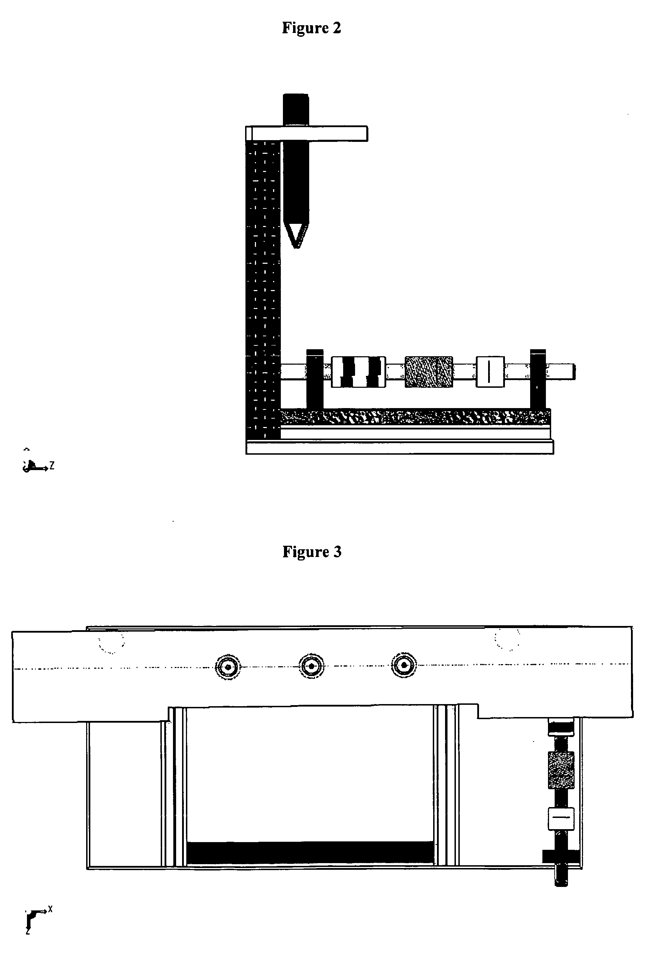

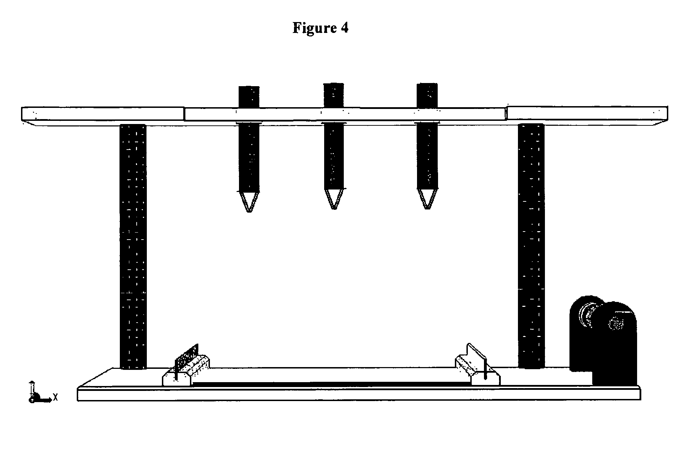

Fig. 2-4 Examples of the preferred apparatus for producing a biocompatible material according to the invention shown from different views.

## Claims

1. A sheet or tubular structure, which consists of biocompatible material, is elastic and comprises at least one biologically active substance in at least one region.

2. A sheet or tubular structure, which consists of biocompatible material, is elastic and comprises at least one biologically active substance release-modifying agent in at least one region.

3. The sheet or tubular structure of claim 1, further comprising a biologically active substance release-modifying agent.

4. The sheet or tubular structure of any of claims 1-3, wherein in the tubular structure has at least one sealed end.

5. The sheet or tubular structure of any of claims 1, 3 or 4, wherein the biocompatible material is capable of locally releasing said biologically active substance under physiological conditions.

6. The sheet or tubular structure of any of claims 1-5, wherein the biocompatible material comprises, essentially consists or consists of (i) a fabric comprising weaves, knits, braids, filament windings, spun windings, a felt, and combinations thereof and/or (ii) a membrane or a foil.

7. The sheet or tubular structure of claim 6, wherein the fabric comprises, essentially consists or consists of fibers of natural polymers, fibers of artificial polymers, fibers of mixed natural and artificial polymers and mixtures thereof.

8. The sheet or tubular structure of claim 7, wherein (i) the natural polymers are selected from the group consisting of silk, cotton, cellulose, agarose, methylcellulose, hyluronan and derivatives thereof and (ii) the artificial polymers are selected from the group consisting of poly(ethylene oxide) (PEO), polytetrafluoroethylene (PTFE), polyethylene terephthalate (PET), polyvinyl alcohol, acrylate polymers, silicone and derivatives thereof.

9. The sheet or tubular structure of any of claims 1-8, wherein the biocompatible material comprises, essentially consists or consists of a hydrogel.

10. The sheet or tubular structure of any of claims 1-9, wherein the biocompatible material is tearproof.

11. The sheet or tubular structure of any of claims 1-10, wherein the biocompatible material is capable of absorbing and/or adsorbing the biologically active substance.

12. The sheet or tubular structure of any of claims 1-11, wherein the biocompatible material is bioresorbable.

13. The tubular structure of any of claims 1-12, wherein the tubular structure has a diameter of at least 0.2 cm.

14. The sheet or tubular structure of any of claims 1-13, wherein the biocompatible material has a length of at least 50 cm.

15. The sheet or tubular structure of any of claims 1, 3-14, wherein the biologically active substance is selected from the group consisting of an adrenocorticostatic, a β-adrenolytic, an androgen or antiandrogen, an antianemic, an antiparasitic, an anabolic, an anaesthetic or analgesic, an analeptic, an antiallergic, an antiarrhythmic, an antiarterosclerotic, an antibiotic, an antidiabetic, an antifibrinolytic, an anticonvulsive, an angiogenesis inhibitor, an anticholinergic, an enzyme, a coenzyme or a corresponding inhibitor, an antihistaminic, an antihypertensive, an antihypotensive, an anticoagulant, an antimycotic, an antiseptic, an antiinfective, an antihemorrhagic, a beta-receptor and calcium channel antagonist, an antimyasthenic, an antiphlogistic, an antipyretic, an antirheumatic, an antiseptic, a cardiotonic, a chemotherapeutic, a coronary dilatator, a cytostatic, a glucocorticoid, a haemostatic, an immunoglobuline or its fragment, a chemokine, a cytokine, a prodrug of a cytokines, a mitogen, a physiological or pharmacological inhibitor of mitogens, a cell differentiation factor, a cytotoxic agent and prodrugs thereof, a hormone, an immunosuppressant, an immunostimulant, a mineralcorticoid, a morphine-antagonist, a muscle relaxant, a narcotic, a vector, a peptide, a (para)-sympathicomimetic or (para)-sympatholytic, a protein, a cell, a selective estrogen receptor modulator (SERM), a sedating agent, a spasmolytic, a substance that inhibits the resorption of bone, a vasoconstrictor or vasodilatator, a virustatic, a wound-healing substance, and combinations thereof.

16. The sheet or tubular structure of claim 15, wherein the vector is a polynucleotide.

17. The sheet or tubular structure of claim 15, wherein the cell is a selected from the group consisting of a mesenchymal cell, an embryonic stem cell, an osteoblast, an osteoclast, a fibroblast and a platelet.

18. The sheet or tubular structure of claim 15, wherein the selective estrogen receptor modulator (SERM) is selected from the group consisting of clomifene, raloxifene, tamoxifen, toremifene, bazedoxifene, lasofoxifene and ormeloxifene.

19. The sheet or tubular structure of claim 15, wherein the substance that inhibits the resorption of bone is selected from the group consisting of strontium ranelate, teriparatide and bisphosphonate.

20. The sheet or tubular structure of claims 15, wherein the protein is matrix metalloproteinase (MMP) or bone morphogenetic protein (BMP).

21. The sheet or tubular structure of claim 15, wherein the antibiotic is selected from the group consisting of an aminoglycoside, an ansamycin, a carbacephem, a carbapenem, a cephalosporin, a macrolide, a monobactam, a tetracycline, an oxalidinone, a beta-lactam antibiotic, a quinolone, a sulfonamide, a rifamycin, a glycopeptide antibiotic, a nitrofurantoin, a fusidic acid, or a pharmaceutically acceptable salt thereof.

22. The sheet or tubular structure of claim 15, wherein the antibiotic is selected from the group consisting of gentamycin, kanamycin, netilmicin, penicillin, streptomycin, amikacin, tobramycin, chloramphenicol, erythromycin lincomycin, rifampin, tetracycline, doxycycline, chlortetracycline, minocycline, linezoloid, penicillin, metronidazole, cephalosporin, dicloxacillin, carbapenem, ampicillin, cefepime, ceftazidime, cefotaxime, cefuroxime, cefaclor, cetriaxone, imipenem, meropenem, ciprofloxacin, moxifloxacin, levofloxacin, sulfamethoxazole, vancomycin and derivatives thereof.

23. The sheet or tubular structure of claims 2-22, wherein the biologically active substance release-modifying agent is selected from serum, platelets, co-solvents, viscosity modifiers, chaotropic agents, polymeric salts, surfactants, polymeric acids, polymeric bases, gels, non-volatile monoprotic or polyprotic organic acid, a non-volatile, mono- or poly-functional base, citric acid and combinations thereof.

24. The sheet or tubular structure of any of claims 2-23, wherein the biologically active substance release-modifying agent comprises a reactive group capable of covalently binding at least one biologically active substance.

25. The sheet or tubular structure of any of claims 3-24, wherein at least one biologically active substance is bound to the biologically active substance release-modifying agent through a covalent bond.

26. The sheet or tubular structure of claims 24 or 25, wherein the biologically active substance release-modifying agent further comprises a biocleavable spacer and/or wherein the bond between the biologically active substance release-modifying agent and the biologically active substance and/or a bond between the biologically active substance release-modifying agent and the biocompatible material is biocleavable.

27. The sheet or tubular structure of any of claims 1-26, wherein the region comprising the biologically active substance and/or the biologically active substance release-modifying agent extends over the entire biocompatible material.

28. An implant covered at least partially by a sheet or tubular structure of any of claims 1 to 27.

29. The implant of claim 28 wherein the implant is an orthopedic implant, an implantable biosensor, an implantable drug infusion tube, an intravitreal drug delivery device, a nerve regeneration conduit, a pacemaker, implantable electrostimulation leads, a spinal repair device, or a stent.

30. A method of producing the sheet or tubular structure of claims 1-27 comprising the step of contacting the sheet or tubular structure in at least one region with at least one biologically active substance and/or at least one biologically active substance release-modifying agent.

31. The method of producing the sheet or tubular structure of claim 30, wherein the biocompatible material is contacted with the biologically active substance release-modifying agent prior to, concomitantly or after contacting the biocompatible material with the biologically active substance.

32. The method of producing the sheet or tubular structure of claims 30 or 31, wherein the contacting is effected by dripping at least one biologically active substance onto at least one region of the sheet or tubular structure.

33. A method of producing an enhanced implant comprising the steps of the method of claims 30-32, and further comprising the step of at least partially covering an implant with the sheet or tubular structure.

34. A sheet or tubular structure producible by the method of claims 30-32.

35. An enhanced implant producible by the method of claim 33.

36. Use of a sheet or tubular structure according to any of claims 1 and 3-27 or the enhanced implant according to claim 35, for the treatment of a disease or an ailment selected from the group consisting of a defective, fractured or lost bone, bone-structure or tooth, of a disease-induced localized flow constriction, of a heart disease, of chronic or acute pain, of a genetic disorder, of arthritis, of rheumatism of cancer of an inflammation and of an infection.

37. Apparatus for producing a sheet or tubular structure according to claims 1-27 comprising:
(i) a dispenser device (2) for dispensing one or more sheets or tubular structures (1);
(ii) at least one reservoir (3) capable of containing a biologically active substance and/or a biologically active substance release-modifying agent; and/or
(iii) a release system (4) capable of releasing a biologically active substance and/or a biologically active substance release-modifying agent from at least one reservoir (3) onto the sheet or tubular structure (1).

38. The apparatus according to claim 37 further comprising at least one cutting device (5) capable of cutting the sheet or tubular structure (1).

39. The apparatus according to claims 37 or 38, wherein the cutting device cuts thermally and/or mechanically.

40. The apparatus according to claims 37-39, wherein the cutting device is capable of sealing and optionally cutting the tubular structure.

41. The apparatus according to any of claims 37-40, wherein the apparatus further comprises a wetting tray (6).

42. The apparatus according to any of claims 37-41, further comprising a refrigeration unit configured to cool at least one reservoir (3).

43. The apparatus according to any of claims 37-42, wherein the dispenser device (2) comprises one or more rolls of a sheet or tubular structure.

44. The apparatus according to any of claims 37-43, wherein the apparatus further comprises a measuring device for measuring the length of the sheet or tubular structure (1) dispensed from the dispenser device (2).

45. The apparatus according to any of claims 37-44, wherein at least one reservoir (3) comprises a controlled release system which permits the release of a specified volume of the biologically active substance and/or a biologically active substance release-modifying agent from the reservoir (3) onto the sheet or tubular structure (1).

46. The apparatus according to any of claims 37-45, wherein the controlled release system further comprises an indicator indicating the volume and/or amount and/or costs of the biologically active substance and/or a biologically active substance release-modifying agent which is to be released from the reservoir (3).

47. Method of treating a patient suffering from a disease or an ailment selected from the group consisting of a defective, fractured or lost bone, bone-structure or tooth, disease-induced localized flow constriction, heart disease, chronic or acute pain, genetic disorder, arthritis, rheumatism, cancer, inflammation and infection, wherein the method of treating a patient comprises the steps:
(i) using the apparatus according to any of claims 37-46 to produce a customized sheet or tubular structure according to claims 1-25; wherein the sheet or tubular structure is preferably customized to meet the needs of the patient;
(ii) optionally at least partially covering an implant with the customized sheet or tubular structure; and
(iii) implanting said customized sheet or tubular structure or the implant which is at least partially covered with the customized sheet or tubular structure into the patient.
